# EUROPÄISCHE PATENTANMELDUNG

(11) **EP 2 180 317 A1**
(43) Veröffentlichungstag der Anmeldung: **28.04.2010**
(21) Anmeldenummer: 08018645.5
(22) Anmeldetag: 24.10.2008
(51) Int. Cl.: G01N 27/49, G01F 1/64, G01F 1/704

(54) **System zur Messung einer Analytkonzentration und Verfahren zur Überwachung eines Flüssigkeitsstroms**

(71) Anmelder: Roche Diagnostics GmbH, 68305 Mannheim (DE); F.HOFFMANN-LA ROCHE AG, 4070 Basel (CH)
(72) Erfinder: Kölker, Karl-Heinz, D-67269 Grünstadt (DE); Ocvirk, Gregor, D-55283 Nierstein (DE)
(74) Vertreter: Mommer, Niels

(57) **Zusammenfassung**

Die Erfindung betrifft ein System zur Messung einer Analytkonzentration in einer Flüssigkeit, mit einer Durchflusszelle (3), einem elektrochemischen Sensor (4), der in der Durchflusszelle (3) angeordnet ist und durch Umsetzung des Analyten ein Messsignal (B) erzeugt, einer Verdrängerpumpe (2), um die zu untersuchende Flüssigkeit durch die Durchflusszelle (3) zu pumpen, und einer Steuereinrichtung (7) zur Steuerung der Verdrängerpumpe (2). Erfindungsgemäß ist vorgesehen, dass die Steuereinrichtung (7) das von dem Sensor (4) erzeugte Messsignal (B) auswertet und anhand von darin auftretenden Extrema einen Kontrollwert berechnet, der mit dem Flüssigkeitsstrom durch die Durchflusszelle (3) korreliert, prüft, ob der Kontrollwert außerhalb eines Sollwertebereiches liegt, und falls dies der Fall ist ein Warnsignal erzeugt. Die Erfindung betrifft ferner ein entsprechendes Verfahren sowie die Verwendung eines elektrochemischen Sensors (4) zur Strömungsmessung.

## Beschreibung

Die Erfindung geht aus von einem System zur Messung einer Analytkonzentration in einer Flüssigkeit wie es beispielsweise in der WO 2005/026665 beschrieben ist. Derartige Systeme haben eine Durchflusszelle, in der ein elektrochemischer Sensor angeordnet ist, und eine Pumpe, um eine zu untersuchende Flüssigkeit durch die Durchflusszelle zu pumpen.

Solche Messsysteme können beispielsweise als so genannte Mikrodialysesysteme ausgebildet sein, bei denen eine Perfusionsflüssigkeit durch eine in Körpergewebe implantierte Mikrodialysesonde hindurch gepumpt wird, so dass Analytmoleküle aus umgebendem Körpergewebe und/oder Körperflüssigkeit in die Perfusionsflüssigkeit hinein diffundieren. Im Idealfall wird auf diese Weise erreicht, dass sich in der Perfusionsflüssigkeit dieselbe Analytkonzentration wie in dem die Sonde umgebenden Körpergewebe einstellt. Mit einem Sensor des Mikrodialysesystems kann dann die Analytkonzentration in der Perfusionsflüssigkeit gemessen und somit auch die Analytkonzentration in Körpergewebe bzw. Körperflüssigkeit des Patienten bestimmt werden. Ein Mikrodialysesystem ist in der EP 1 880 669 A1 beschrieben.

Wichtigste Anwendung von Mikrodialysesystemen ist die Messung der Glukosekonzentration zur Diabetesbehandlung. Die dafür verwendeten Messsysteme müssen klein und leicht sein, da sie von Diabetikern ständig mitgeführt werden. Prinzipiell ist es zur kontinuierlichen Messung des Glukosegehalts mit einem tragbaren Messsystem aber nicht erforderlich, dass eine Perfusionslösung durch eine Mikrodialysesonde hindurch gepumpt wird. Möglich ist es beispielsweise auch, mit einer Sonde kontinuierlich eine geringe Menge Körperflüssigkeit aus Körpergewebe zu entnehmen und mit einem Sensor die Konzentrationsbestimmung unmittelbar an der Körperflüssigkeit vorzunehmen.

Bei Systemen der eingangs genannten Art ist es wichtig, dass eventuelle Fehlfunktionen möglichst frühzeitig erkannt werden. Deshalb sind bei bekannten Systemen üblicherweise mehr oder weniger aufwendige Steuer- oder Kontrolleinrichtungen vorgesehen. Zusätzliche Kontrollfunktionen zur Überwachung eines Messsystems führen in der Regel aber zu höheren Kosten, einem höheren Energieverbrauch und einem größeren Gewicht, was insbesondere bei tragbaren Messsystemen nachteilig ist. Überdies müssen zusätzliche Steuer- und Kontrolleinrichtungen aufwändig kalibriert und typischerweise nach jeder Anwendung sterilisiert werden.

Aufgabe der vorliegenden Erfindung ist es deshalb, einen Weg aufzuzeigen, wie bei einem System zur Messung einer Analytkonzentration in einer Flüssigkeit mit geringem Aufwand eine bessere Kontrolle möglich ist und eventuelle Fehlfunktionen möglichst frühzeitig erkannt werden können.

Diese Aufgabe wird durch ein System mit den im Anspruch 1 angegebenen Merkmalen gelöst. Die Aufgabe wird ferner durch ein Verfahren zur Überwachung eines Flüssigkeitsstroms in einer Durchflusszelle mit den im Anspruch 7 angegebenen Merkmalen sowie die Verwendung eines elektrochemischen Sensors zur Strömungsmessung, der durch Umsetzung eines Analyten in einem ersten Schritt ein Reaktionsprodukt, beispielsweise Wasserstoffperoxid, erzeugt, das in einem weiteren Schritt zur Erzeugung eines elektrischen Signals elektrochemisch umgesetzt wird, gelöst. Vorteilhafte Weiterbildungen der Erfindung sind Gegenstand der Unteransprüche.

Im Rahmen der Erfindung wurde erkannt, dass die üblicherweise bei fluidischen Messsystemen eingesetzten Verdrängerpumpen in dem Messsignal eines elektrochemischen Sensors reproduzierbar Extrema verursachen, die in der Regel periodisch gemäß dem Arbeitszyklus der Verdrängerpumpe auftreten. Anstatt diese Extrema als unerwünschte Störung des Messsignals zu betrachten, welche die präzise Bestimmung der Analytkonzentration erschweren, haben die Erfinder erkannt, dass diese Extrema eine Kontrolle der Arbeitsweise des Systems ermöglichen. Die in dem Messsignal auftretenden Extrema lassen nämlich Rückschlüsse auf den durch die Durchflusszelle fließenden Flüssigkeitsstrom zu, so dass beispielsweise Lecks oder eine Blockade der Flüssigkeitsleitung sowie eine Fehlfunktion der Pumpe erkannt werden können.

Verdrängerpumpen haben einen Arbeitsraum, der sich abwechselnd vergrößert und verkleinert, so dass sich ein Flüssigkeitsstrom ergibt, der mit dem Arbeitstakt der Pumpe pulsiert. Bekannte Formen von Verdrängerpumpen sind beispielsweise Balgpumpen, Membranpumpen, Kolbenpumpen und Peristaltikpumpen. Diese Beispiele zeigen, dass Verdrängerpumpen in vielen unterschiedlichen Ausführungsformen bekannt sind. Ein sehr bekanntes Beispiel einer Verdrängerpumpe ist auch das menschliche Herz.

Verdrängerpumpen bewirken einen pulsierenden Flüssigkeitsstrom, also einen Flüssigkeitsstrom, dessen Strömungsgeschwindigkeit durch aufeinander folgende Extrema gekennzeichnet ist. Diese Extrema des Flüssigkeitsstroms können entsprechende Extrema im Messsignal eines elektrochemischen Sensors bewirken, so dass sich ein elektrochemischer Sensor als Strömungssensor verwenden lässt. Die erfindungsgemäß ausgewerteten Extrema im Messsignal können Minima oder Maxima sein. Möglich ist es auch, sowohl Minima als auch Maxima auszuwerten.

Da ein elektrochemischer Sensor, beispielsweise ein amperometrischer Sensor, zur Messung der Analytkonzentration der Flüssigkeit ohnehin benötigt wird, ermöglicht die vorliegende Erfindung ohne den Aufwand eines zusätzlichen Sensors und ohne Erhöhung des Energieverbrauchs eine Kontrolle des Flüssigkeitsstroms des Messsystems. Erfindungsgemäß kann deshalb ohne zusätzlichen apparativen Aufwand oder Erhöhung des Gesamtgewichts des Messsystems ein zu hoher oder zu niedriger Flüssigkeitsstrom, der beispielsweise auf einer Fehlfunktion der Pumpe oder einer störungsbedingten Verengung eines Leitungsquerschnitts beruht, frühzeitig erkannt und ein entsprechendes Warnsignal erzeugt werden. Mit einem solchen Warnsignal kann ein Benutzer auf ein Problem aufmerksam gemacht werden, so dass geeignete Gegenmaßnahmen ergriffen werden können. Möglich ist es auch, dass ein solches Warnsignal zu einem Abschalten der Pumpe führt oder in anderer Weise als Steuerungssignal verwendet wird.

Zur Überwachung eines Messsystems kann beispielsweise anhand des zeitlichen Abstandes aufeinander folgender Extrema des Messsignals berechnet werden, wie lange ein Arbeitszyklus der Pumpe dauert, also beispielsweise die Drehzahl der Pumpe berechnet werden. Ergibt sich dabei, dass die Dauer eines Arbeitszyklus bzw. die Drehzahl der Pumpe um mehr als eine zulässige Toleranz von einem Sollwert abweichen, so deutet dies auf eine Fehlfunktion des Systems hin.

Eventuelle Fehlfunktionen eines Messsystems können beispielsweise auch durch eine Auswertung der Stärke der Extrema erkannt werden, beispielsweise indem eine Abweichung der Extrema von einem Untergrundsignal, also der Höhe eines Maximums bzw. der Tiefe eines Minimums, untersucht werden. Insbesondere kann ein Kontrollwert, der mit dem Flüssigkeitsstrom korreliert, als eine Funktion der Abweichung der Extrema von einem Durchschnittssignal berechnet werden. Ist nämlich der Auslass der Durchflusszelle, in welcher der Sensor angeordnet ist, durch eine Störung verkleinert oder eine dahinter liegende Flüssigkeitsleitung teilweise blockiert, so führt dies zu einem erhöhten Strömungswiderstand und insgesamt zu einer Veränderung der Extrema des Sensorsignals.

Die Höhe eines Maximums bzw. die Tiefe eines Minimums kann beispielsweise in Bezug auf einen Durchschnittswert des Messsignals zwischen aufeinander folgenden Minima bzw. Maxima berechnet werden. Eine weitere Möglichkeit ist, den Unterschied zwischen der Höhe eines Maximums und der Tiefe eines darauf folgenden Minimums zu verwenden. Möglich ist es auch, einen Kontrollwert anhand der Breite der Extrema zu berechnen.

Ein erfindungsgemäßes Verfahren lässt sich prinzipiell auch bei einem elektrochemischen Sensor in Kombination mit einer Strömungs- oder Kreiselpumpe, die an sich einen kontinuierlichen Flüssigkeitsstrom erzeugen könnte, nutzen, sofern durch eine geeignete Pumpensteuerung zu Kontrollzwecken eine Pulsation des Flüssigkeitsstroms bewirkt wird.

Ein erfindungsgemäß verwendeter Sensor kann durch Umsetzung des Analyten in einem ersten Schritt ein in der Flüssigkeit bewegliches Reaktionsprodukt, beispielsweise Wasserstoffperoxid, erzeugen, das in einem weiteren Schritt von dem Elektrodensystem des Sensors elektrochemisch ungesetzt wird, so dass ein elektrisches Signal, beispielsweise ein elektrischer Strom, entsteht. Dieser elektrische Strom ist im Allgemeinen umso stärker, je mehr Analytmoleküle umgesetzt werden, also je höher die Analytkonzentration ist. Das bewegliche Reaktionsprodukt kann durch eine chemische, insbesondere eine enzymatische, Reaktion erzeugt werden, an welcher der Analyt beteiligt ist. Bevorzugt wird erfindungsgemäß ein enzymatischer Sensor verwendet, also ein Sensor, der eine enzymkatalytische Umsetzung des Analyten bewirkt. Bevorzugt hat der verwendete Sensor zusätzlich zu einer Arbeitselektrode und einer Gegenelektrode auch eine Referenzelektrode.

Die Eignung eines elektrochemischen Sensors zur Strömungsmessung wird darauf zurückgeführt, dass ein Anteil des bei der Umsetzung entstehenden Reaktionsprodukts, beispielsweise Wasserstoffperoxid, von der Flüssigkeitsströmung weggespült werden kann, bevor durch elektrochemische Umsetzung des Reaktionsprodukts an einer Elektrode des Sensors Ladungsträger freigesetzt werden können. Ein stärkerer Flüssigkeitsstrom hat deshalb ein etwas geringeres Messsignal zur Folge, da etwas mehr Moleküle des Reaktionsprodukts weggespült werden und folglich zu dem Messsignal nichts beitragen. Umgekehrt führt ein geringerer Flüssigkeitsstrom zu einem etwas höheren Messsignal, da ein geringerer Anteil des Reaktionsproduktes weggespült und somit ein größerer Anteil elektrochemisch umgesetzt wird. Besonders stark ausgeprägte Extrema können in dem Messsignal des Sensors auftreten, wenn die Rate der elektrochemischen Umsetzung des Reaktionsproduktes in der selben Größenordnung liegt wie die Rate, mit der Moleküle des Reaktionsproduktes weggespült werden.

Das Elektrodensystem eines elektrochemischen Sensors kann mit einer Deckschicht versehen werden. Auf diese Weise lässt sich beeinflussen, wie groß der Anteil des Reaktionsproduktes ist, der bei einer gegebenen Strömungsgeschwindigkeit weggespült wird. Die Durchlässigkeit einer solchen Deckschicht kann nämlich in geeigneter Weise gewählt werden, um den Sensor an die erwarteten Strömungsverhältnisse anzupassen. Auf diese Weise lässt sich der erfindungsgemäß genutzte Effekt einer Abhängigkeit des Messsignals von den Strömungsverhältnissen optimieren, ohne die Messung der Analytkonzentration wesentlich zu erschweren.

Weitere Einzelheiten und Vorteile der Erfindung werden an einem Ausführungsbeispiel unter Bezugnahme auf die beigefügten Zeichnungen erläutert. Es zeigen:
- Figur 1: eine schematische Darstellung eines Ausführungsbeispiel eines erfindungsgemäßen Systems zur Messung einer Analytkonzentration in einer Flüssigkeit;
- Figur 2: eine weitere schematische Darstellung des in Figur 1 gezeigten Messsystems; und
- Figur 3: ein Beispiel eines Messsignals des elektrochemischen Sensors des dargestellten Systems sowie der mit einem herkömmlichen Flussratensensor zum Vergleich gemessenen Flussrate.

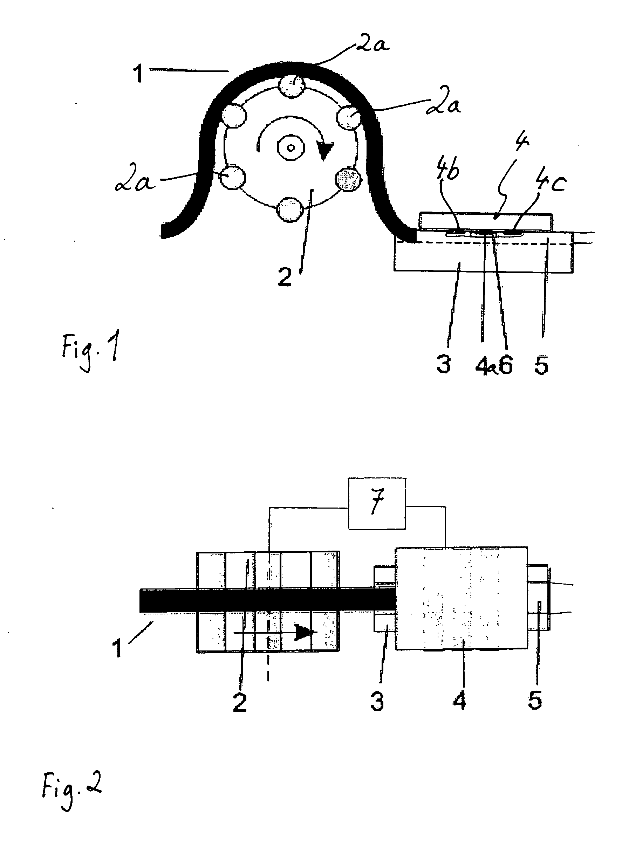

Die Figuren 1 und 2 zeigen schematisch in unterschiedlicher Ansicht ein Ausführungsbeispiel eines System zur Messung einer Analytkonzentration in einer Flüssigkeit. Das dargestellte Messsystem hat eine Flüssigkeitsleitung 1, durch die mittels einer Pumpe 2 Flüssigkeit zu einer Durchflusszelle 3 gepumpt wird, in der ein elektrochemischer Sensor 4 angeordnet ist, der durch enzymkatalytische Umsetzung des Analyten ein Messsignal erzeugt. Die Pumpe 2 wird von einer Steuereinrichtung 7 gesteuert, die an den Sensor 4 angeschlossen ist. Im Betrieb erhält die Steuereinrichtung 7 von dem Sensor 4 erzeugte Messsignale zur Auswertung.

Bei dem dargestellten Ausführungsbeispiel ist die Pumpe 2 eine Verdrängerpumpe, nämlich eine Peristaltikpumpe, die mit Quetschelementen 2a auf einen als Schlauch ausgebildeten Abschnitt der Flüssigkeitsleitung 1 einwirkt, wobei selbstverständlich auch andere Pumpen verwendet werden können.

Der elektrochemische Sensor 4 hat ein Elektrodensystem, das zumindest eine Arbeitselektrode 4a und eine Gegenelektrode 4b, bevorzugt auch eine Referenzelektrode 4c, aufweist. Die Arbeitselektrode 4a weist eine Schicht auf, in der Enzymmoleküle, beispielsweise eine Oxidase, immobilisiert sind, beispielsweise durch eine chemische Bindung oder eine Diffusionsbarriere. Analytmoleküle, beispielsweise Glukose oder Laktat, können in der enzymhaltigen Schicht in einem ersten Schritt einer Nachweisreaktion enzymkatalytisch mit einem passenden Enzym, also beispielsweise einer Glucosoxidase bzw. einer Laktaseoxidase, umgesetzt werden. Dabei entsteht ein in der Flüssigkeit bewegliches Reaktionsprodukt, beispielsweise Wasserstoffperoxid, das in einem weiteren Schritt an der Arbeitselektrode 4a des Sensors 4 elektrochemisch umgesetzt wird, so dass Ladungsträger frei werden, die ein elektrisches Messsignal bewirken. Bevorzugt ist dieses Messsignal ein elektrischer Strom, der in der Regel umso stärker ist, je höher die Analytkonzentration in der Flüssigkeit ist. Als elektrisches Messsignal kann jedoch die elektrische Spannung oder der elektrischen Widerstands des Sensors ausgewertet werden.

Das Elektrodensystem des Sensors 4 ist von einer Deckschicht 6 bedeckt, die für Analytmoleküle und das Reaktionsprodukt, also beispielsweise Glukose und Wasserstoffperoxid, durchlässig ist.

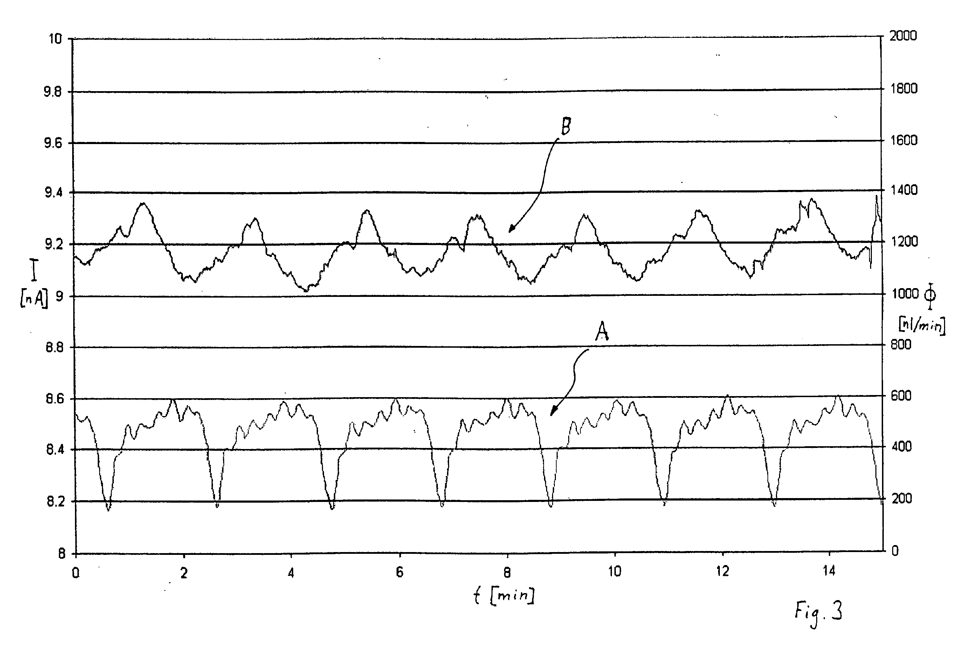

Der Arbeitstakt der Verdrängerpumpe 2 bewirkt Pulsationen des durch die Durchflusszelle 3 in eine anschließende Leitung 5 fließenden Flüssigkeitsstroms. Diese Pulsationen wurden bei dem dargestellten Ausführungsbeispiel mit einem herkömmlichen Strömungssensor gemessen und sind in Figur 3 als Signal A über der Zeit t in Minuten dargestellt. Das Signal A gibt den Flüssigkeitsstrom φ in Nanoliter pro Minute gemäß der rechten Skale an. In Figur 3 ist zusätzlich das von dem elektrochemischen Sensor 4 als Strom I erzeugte Messsignal B in Nanoampere über der Zeit t in Minuten dargestellt.

In Figur 3 ist deutlich zu erkennen, dass das Messsignal B des elektrochemischen Sensors 4 ausgeprägte Extrema, nämlich Minima, zeigt, die in Abständen von etwa zwei Minuten auftreten und mit den Extrema des Flüssigkeitsstroms korrelieren. Dies wird darauf zurückgeführt, dass bei hoher Strömungsgeschwindigkeit ein größerer Anteil des von dem Sensor 4 durch enzymkatalytische Umsetzung des Analyten erzeugten Reaktionsproduktes Wasserstoffperoxid fortgespült wird ohne zu dem Messsignal beitragen zu können. Umgekehrt wird bei kleinerer Strömungsgeschwindigkeit ein geringerer Anteil des Reaktionsproduktes weggespült, so dass sich ein entsprechend größerer Strom I als Messsignal ergibt.

Durch Auswertung der Extrema lässt sich deshalb der Flüssigkeitsstrom in der Durchflusszelle 3 überwachen, also der elektrochemische Sensor 4 als Strömungssensor verwenden. Mit der Steuereinrichtung 7 wird dazu das von dem Sensor 4 erzeugte Messsignal B ausgewertet und anhand von darin auftretenden Extrema ein Kontrollwert berechnet, der mit dem Flüssigkeitsstrom durch die Durchflusszelle 3 korreliert.

Der Kontrollwert kann beispielsweise aus dem zeitlichen Abstand zwischen den einzelnen Extrema des Messsignals B berechnet werden. Bei einer gleichmäßig laufenden Verdrängerpumpe treten Pulsationen des Flüssigkeitsstromes und folglich auch die Extrema nämlich periodisch auf. Aus dem zeitlichen Abstand zwischen den einzelnen Extrema kann deshalb die Drehzahl der Pumpe 2 berechnet werden. Eine zu große oder zu kleine Drehzahl kann so anhand des Kontrollwertes erkannt und durch ein Warnsignal angezeigt werden.

Bei dem dargestellten Ausführungsbeispiel hat die Peristaltikpumpe 2 insgesamt sechs Quetschelemente 2a, so dass bei einem kompletten Umlauf der Peristaltikpumpe 2 insgesamt sechs Maxima der Strömungsgeschwindigkeit auftreten. Die Zeitspanne, in der sechs Maxima des Messsignals B auftreten, entspricht deshalb der Zeit eines kompletten Umlaufs der Peristaltikpumpe 2. Aus den Charakteristika der Pumpe 2, beispielsweise der Anzahl der Quetschelemente 2a einer Peristaltikpumpe, lässt sich somit durch Auswertung des zeitlichen Abstandes zwischen den einzelnen Extrema die Drehzahl der Pumpe 2 berechnen.

Ein Kontrollwert kann auch durch Auswertung der Stärke der Extrema berechnet werden. Eine Erhöhung des Strömungswiderstandes, beispielsweise wegen einer störungsbedingten Verengung des Leitungsquerschnitts, führt dazu, dass sich die periodisch auftretenden Extrema des Messsignals B verändern. Als Kontrollwert kann auf diese Weise beispielsweise der Unterschied zwischen einem Maximum und einem darauf folgenden Minimum oder die Abweichung eines Extremums von einem Durchschnittswert berechnet werden. Durch Vergleich des Kontrollwertes mit einem Sollwert können so Störungen des Flüssigkeitsstroms, beispielsweise Lecks oder eine Verstopfung der Flüssigkeitsleitung, erkannt werden. Die Steuereinrichtung 7 prüft hierfür, ob der Kontrollwert außerhalb eines Sollwertebereiches liegt, und erzeugt, falls dies der Fall ist, ein Warnsignal.

Ein Kontrollwert kann dabei auch unter Berücksichtung sowohl der zeitlichen Abstände aufeinander folgender Extrema als auch der Stärke der Extrema berechnet werden. Beispielsweise kann ein Kontrollwert als ein mehrdimensionaler Wert, insbesondere in Form eines Vektors berechnet werden, der als ersten Eintrag einen Kontrollwert zur Drehzahl der Pumpe und als zweiten Eintrag einen als Funktion der Stärke der Extrema berechneten Wert enthält.

Die Steuereinrichtung des Flüssigkeitssystems prüft, ob ein berechneter Kontrollwert außerhalb eines Sollwertebereichs liegt und erzeugt, falls dies der Fall ist, ein Warnsignal. Mit einem solchen Warnsignal kann ein Benutzer auf eine Störung des Systems aufmerksam gemacht werden. Möglich ist es auch, dass dieses Warnsignal als Steuerungssignal verwendet wird, beispielsweise um die Pumpe abzuschalten oder ihre Drehzahl zu regeln.

Das schematisch in den Figuren 1 und 2 dargestellte Messsystem kann beispielsweise zur kontinuierlichen Überwachung der Glukosekonzentration in einer Körperflüssigkeit eines Diabetikers verwendet werden. Hierzu kann beispielsweise die Flüssigkeitsleitung 1 an eine Sonde angeschlossen werden, mit der ständig eine kleine Menge Körperflüssigkeit entnommen wird. Der Ausgang der Leitung 5 wird in einem solchen Fall an ein Abfallreservoir angeschlossen, das die Körperflüssigkeit nach einer Messung aufnimmt. Möglich ist es auch, das Messsystem als Teil eines Mikrodialysesystems zu nutzen und die Flüssigkeitsleitung 1 hierfür an einen Perfusionsflüssigkeitsreservoir anzuschließen. Zwischen der Pumpe und der Durchflusszelle 3 kann die Flüssigkeit dann durch eine Sonde hindurch und anschließend zu der Durchflusszelle 3 geleitet werden. Bei einem solchen Perfusionssystem kann es vorteilhaft sein, der Flüssigkeit zwischen der Sonde und der Durchflusszelle eine weitere Flüssigkeit zu zumischen.

Die Sonde eines Mikrodialysesystems wird bestimmungsgemäß im Körpergewebe eines Patienten eingestochen und weist eine Hin- und eine Rückleitung für die Perfusionsflüssigkeit auf. Beim Durchströmen der Sonde nimmt die Perfusionsflüssigkeit Analytmoleküle aus Körperflüssigkeit oder Körpergewebe, das die Sonde umgibt, auf. Die Leitung 5 wird auch in einem solchen Fall an ein Abfallreservoir zur Aufnahme verbrauchter Perfusionsflüssigkeit angeschlossen.

Wird das in den Figuren 1 und 2 dargestellte Messsystem als Mikrodialysesystem verwendet, ist eine Flussrate von 50 bis 200 nl/min vorteilhaft. Bei der dargestellten Peristaltikpumpe 2 mit sechs Quetschelementen lässt sich dies beispielsweise mit einer Umdrehungszahl von 0,1 bis 0,4 Umdrehungen pro Minute und einem Schlauchdurchmesser von 0,1 mm erreichen. Im allgemeinen ist es vorteilhaft, den Querschnitt der Durchflusszelle 3 größer als den vorhergehenden Leitungsquerschnitt auszubilden. Beispielsweise kann die Durchfusszelle 3 in ihrem Inneren eine Breite von 0,1 bis 0,9 mm und eine Höhe von 0,1 bis 0,2 mm aufweisen. Die Dicke der Deckschicht 6 kann beispielsweise 1 bis 30 µm betragen und einen Diffusionskoeffizienten für das Reaktionsprodukt Wasserstoffperoxid von beispielsweise 5·10⁻⁵ bis 10⁻⁶ cm²/s aufweisen

### Bezugszeichen

- 1: Flüssigkeitsleitung
- 2: Pumpe
- 2a: Quetschelement
- 3: Durchflusszelle
- 4: Elektrochemischer Sensor
- 4a: Arbeitselektrode
- 4b: Gegenelektrode
- 4c: Referenzelektrode
- 5: Leitung
- 6: Deckschicht
- 7: Steuereinrichtung
- A: Vergleichssignal eines Strömungssensors
- B: Messsignal

## Patentansprüche

1. System zur Messung einer Analytkonzentration in einer Flüssigkeit, mit einer Durchflusszelle (3),
einem elektrochemischen Sensor (4), der in der Durchflusszelle (3) angeordnet ist und durch Umsetzung des Analyten ein Messsignal (B) erzeugt,
einer Verdrängerpumpe (2), um die zu untersuchende Flüssigkeit durch die Durchflusszelle (3) zu pumpen, und
einer Steuereinrichtung (7) zur Steuerung der Verdrängerpumpe (2),
**dadurch gekennzeichnet, dass**
die Steuereinrichtung (7) das von dem Sensor (4) erzeugte Messsignal (B) auswertet und anhand von darin auftretenden Extrema einen Kontrollwert berechnet, der mit dem Flüssigkeitsstrom durch die Durchflusszelle (3) korreliert, prüft, ob der Kontrollwert außerhalb eines Sollwertebereiches liegt, und falls dies der Fall ist ein Warnsignal erzeugt.

2. System nach Anspruch 1, **dadurch gekennzeichnet, dass** die Verdrängerpumpe (2) eine Peristaltikpumpe ist.

3. System nach einem der vorstehenden Ansprüche, **dadurch gekennzeichnet, dass** der elektrochemische Sensor (4) durch Umsetzung des zu messenden Analyten Wasserstoffperoxid erzeugt.

4. System nach einem der vorstehenden Ansprüche, **dadurch gekennzeichnet, dass** die Steuereinrichtung (7) den Kontrollwert anhand von periodisch auftretenden Extrema des Messsignals (B) berechnet.

5. System nach einem der vorstehenden Ansprüche, **dadurch gekennzeichnet, dass** die Steuereinrichtung (7) den Kontrollwert anhand von Minima des Sensorsignals berechnet.

6. System nach einem der vorstehenden Ansprüche, **dadurch gekennzeichnet, dass** der Sensor (4) eine Arbeitselektrode (4a) aufweist, die eine von einer Deckschicht (6) bedeckte Schicht aufweist, die Enzymmoleküle enthält.

7. Verfahren zur Überwachung eines Flüssigkeitsstroms in einer Durchflusszelle (3), in der ein elektrochemischer Sensor (4) zur Messung einer Analytkonzentration der durchströmenden Flüssigkeit angeordnet ist, **dadurch gekennzeichnet, dass** ein von dem Sensor (4) durch Umsetzung eines Analyten erzeugtes Messsignal (B) ausgewertet und anhand von darin auftretenden Extrema ein Kontrollwert berechnet wird, der mit dem Flüssigkeitsstrom korreliert, geprüft wird, ob der Kontrollwert außerhalb eines Sollwertebereiches liegt, und falls dies der Fall ist ein Warnsignal erzeugt wird.

8. Verfahren nach Anspruch 7, **dadurch gekennzeichnet, dass** in dem Flüssigkeitsstrom Pulsationen erzeugt werden.

9. Verfahren nach Anspruch 7 oder 8, **dadurch gekennzeichnet, dass** die Flüssigkeit mit einer Verdrängerpumpe (2) durch die Durchflusszelle (3) gepumpt wird.

10. Verfahren nach einem der Ansprüche 7 bis 9, **dadurch gekennzeichnet, dass** der Kontrollwert anhand von periodisch auftretenden Extrema berechnet wird.

11. Verfahren nach einem der Ansprüche 7 bis 10, **dadurch gekennzeichnet, dass** der Kontrollwert anhand von Maxima des Messsignals (B) berechnet wird.

12. Verfahren nach einem der Ansprüche 7 bis 11, **dadurch gekennzeichnet, dass** der Kontrollwert aus dem zeitlichen Abstand zwischen Extrema berechnet wird.

13. Verfahren nach einem der Ansprüche 7 bis 12, **dadurch gekennzeichnet, dass** der Kontrollwert als Funktion der Stärke der Extrema berechnet wird.

14. Verfahren nach einem der Ansprüche 7 bis 13, **dadurch gekennzeichnet, dass** das Warnsignal als Steuersignal zur Pumpensteuerung verwendet wird.

15. Verwendung eines elektrochemischen Sensors (4) zur Strömungsmessung, der durch Umsetzung eines Analyten in einem ersten Schritt ein Reaktionsprodukt erzeugt, das in einem weiteren Schritt zur Erzeugung eines elektrischen Signals elektrochemisch umgesetzt wird.
